(19) Europäisches Patentamt
European Patent Office
Office européen des brevets

(11) Publication number : **0 454 459 A2**

# (12) EUROPEAN PATENT APPLICATION

(21) Application number : **91303730.5**

(51) Int. Cl.[5] : **C07C 317/44,** A61K 31/16

(22) Date of filing : **25.04.91**

(30) Priority : **25.04.90 JP 109550/90**

(43) Date of publication of application :
**30.10.91 Bulletin 91/44**

(84) Designated Contracting States :
**AT BE CH DE DK ES FR GB GR IT LI LU NL SE**

(71) Applicant : **TAISHO PHARMACEUTICAL CO. LTD**
**24-1 Takata 3-chome Toshima-ku**
**Tokyo 171 (JP)**

(72) Inventor : **Tomisawa, Kazuyuki**
**191-3, Sakae 4-chome, Inamachi**
**Kitaadachi-gun, Saitama-ken (JP)**
Inventor : **Kameo, Kazuya**
**17-8, Akamidai-4-chome**
**Konosu-shi (JP)**
Inventor : **Hatada, Yuichi**
**8-17, Minamimagome-4-chome**
**Ota-ku, Tokyo (JP)**
Inventor : **Takahasi, Yuki**
**Koopo Sanraizu 202**
**41-7, Haraichi, Ageo-shi (JP)**
Inventor : **Hatayama, Katsuo**
**Danchi 35-3, 1200-215, Horisakicho**
**Omiya-shi (JP)**

(74) Representative : **Lamb, John Baxter et al**
**MARKS & CLERK 57-60 Lincoln's Inn Fields**
**London WC2A 3LS (GB)**

## (54) Sulfonylalkanoic acid amide derivatives.

(57) Sulfonylaklanoic acid amide derivatives of the formula :

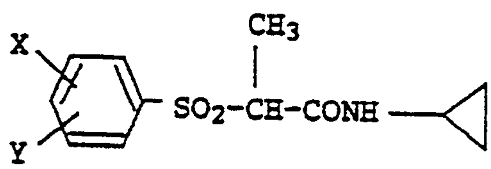

(I)

(wherein X is a halogen atom, and Y is a hydrogen or halogen atom) have good muscle relaxant effects with low side effects.

EP 0 454 459 A2

Jouve, 18, rue Saint-Denis, 75001 PARIS

The present invention relates to novel sulfonylalkanoic acid amide derivatives and a pharmaceutical use of the same for relaxing muscle to tone.

As prior thioalkanoic acid amide derivatives, there are known certain alkanoic acid amide derivatives (Japan Patent Publication No. 63-38026) having anti-ulcer property, arylsulfonylfatty acid amide derivatives as herbicides (Japanese Patent Kokai No. 61-27905) and substituted mercapto acid amides for use in correcting an imbalance of immune homeostasis (U.S. Pat. 4,329,363). However, these properties of the prior art compounds are different from muscle relaxant. No sulfonyloalkanoic acid amide derivatives having muscle relaxing property have been reported in the past.

The dolorous muscle spasm caused by the disorder of motor nervous system (e.g., low-back pain, back pain and herniated disc of the spine) and spastic paralysis such as the cerebral injuries attact an interest in recent years, and there is a need for the appearance of excellent neutrally-acting muscle relaxants for the treatment of the above diseases.

As a result of earnest researches, the present inventors have found some sulfonylalkanoic acid amide derivatives, coming within the compounds of Formula (I) in the specification of the above U.S. Patent but which are not specifically described therein, have much stronger and prolonged muscle relaxing effect with weak side effect, and have accomplished the present invention based on the above finding.

SUMMARY OF THE INVENTION

An object of the present invention is to provide a sulfonylalkanoic acid amide derivative represented by the formula

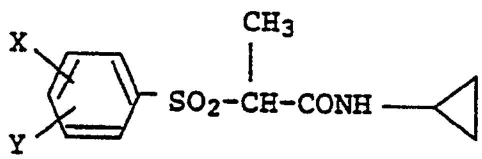

(I)

wherein X is a halogen atom, and Y is a hydrogen atom or a halogen atom.

In another aspect of the present invention, there is provided a pharmaceutical composition for relaxing muscle tone which comprises as an effective ingredient a sulfonylalkanoic acid amide derivative of Formula I and a pharmaceutically acceptable carrier.

In further another aspect of the present invention, there is provided a method for relaxing muscle tone which comprises administering to a patient a pharmaceutically effective amount of the sulfonylalanoic acid amide derivative of Formula (I).

DETAILED EXPLANATION OF THE INVENTION

In the present specification, the halogen atom refers to a fluorine, chlorine, bromine or iodide atom.

Preferred compounds of Formula (I) are N-cyclopropyl-2-(2-bromophenylsulfonyl)propionamide and N-cyclopropyl-2-(2,4-dichlorophenylsulfonyl)propionamide.

The compound of the present invention can be prepared by the following method: a phenylthioalkanoic acid represented by the formula

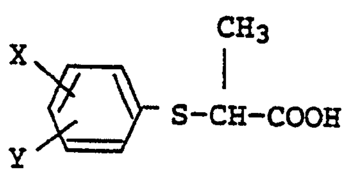

(II)

(wherein X and Y are as defined above) or an activated derivative thereof (e.g., an acid halide and a mixed acid anhydride) is allowed to react with cyclopropylamine according to an usual amidation to give a sulfide compound represented by the formula

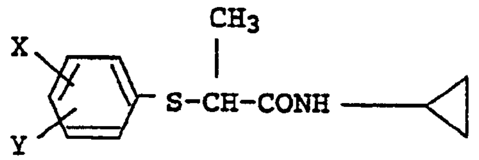

(III)

(wherein X and Y are as defined above), which is then allowed to react with an oxidizing agent to give the compound of Formula (I).

Preferred examples of the amidation are an acid halide method which comprises reacting an acid halide (obtained by reacting the compound of Formula (II) with a halogenating agent) with cyclopropylamine, and a mixed acid anhydride method which comprises reacting a mixed acid anhydride (obtained by reacting a compound of Formula (II) with an alkyl halocarboxylate) with cyclopropylamine.

Examples of the halogenating agent used in the acid halide method are thionyl chloride, phosphorus pentachloride, phosphorus oxychloride, oxalyl chloride, thionyl bromide and phosphorus tribromide.

The compound of Formula (II) and an equimolar or large excess amounts of the halogenating agent are stirred together in the absence or presence of an inert solvent (e.g., benzene, toluene, tetrahydrofuran, methylene chloride and chloroform) at a temperature of from room temperature to the reflux temperature of the solvent for 0.5 to 10 hours to give an acid halide of the compound of Formula (II). In this reaction, the addition of a catalyst is not always essential, but the acceleration of the reaction can be achieved by adding, for example, pyridine, triethylamine and N,N-dimethylformamide in the catalytic amount to an equimolar amount.

The reaction of the acid halide of the compound of Formula (II) with cyclopropylamine can be carried out in the above-mentioned inert solvent. In order to eliminate a side product hydrogen halide obtained in this reaction, the amount of cyclopropylamine is preferably not less than 2 molar amounts. However, when an equimolar amount of cyclopropylamine is used, the reaction may be carried out in the presence of a tertiary amine (e.g., pyridine, triethylamine and N-methylmorpholine) or an alkaline metal base (e.g., sodium hydroxide, potassium hydroxide, potassium carbonate and sodium bicarbonate). This reaction is accomplished at -30 to 50°C for one to 24 hours.

Examples of the alkyl halocarboxylate used in the mixed acid anhydride method are methyl chlorocarboxylate, ethyl chlorocarboxylate, isobutyl chlorocarboxylate and ethyl bromocarboxylate. The compound of Formula (II) and an equimolar to 2 molar amounts of the alkyl halocarboxylate are stirred together in the above-mentioned inert solvent in the presence of a base (e.g., triethylamine, diisopropylamine and N-methylmorpholine) at -30 to 30°C for 0.5 to 3 hours to give a mixed acid anhydride of the compound of Formula (II).

The resulting mixed acid anhydride in the reaction solution as such, without isolation, is allowed to react with cyclopropylamine at the same temperature to give the compound of Formula (III). This reaction is accomplished for 0.5 to 3 hours.

The reaction for preparing the compound of Formula (I) is carried out by reacting the compound of Formula (III) with 2 - 3 molar amounts of an oxidizing agent in an organic solvent (e.g., dichloromethane, chloroform, dichloroethane, benzene, toluene, ethyl acetate and acetic acid) at 0 to 150°C. This reaction is accomplished for one to 70 hours. Examples of the oxidizing agent are hydrogen peroxide and organic peracid (e.g., peracetic acid, trifluoroperacetic acid, perbenzoic acid, perphthalic acid and m-chloroperbenzoic acid).

The compounds of Formula (I) exhibit remarkable muscle relaxation activity and rigidity, mitigation activity. Accordingly, these compounds are useful as the therapeutic agents of the dolorous muscle spasm caused by the disorder of motor nervous system (e.g., low-back pain, back pain and herniated diac of the spine) or spastic paralysis such as the cerebral injuries. For these porposes, the compound of the present invention is mixed with suitable pharmaceutically acceptable carriers for solid or liquid form to give the pharmaceutical preparation for oral or parenteral administration. Examples of the pharmaceutical preparation are solid forms such as tablets, pills, capsules and granules, liquid forms such as injectional solutions, syrups and emulsions, and external forms such as ointments and suppositories, all of which can be prepared according to conventional pharmaceutical practices. The carriers in the above-mentioned preparations can include ordinary additives such as auxiliaries, stabilizers, wetting agents and emulsifiers. For example, there can be used solubilizers (e.g., injectional distilled water, physiological saline solution and Ringer's solution) and preservers (e.g., methyl p-oxybenzoate and propyl p-oxybenzoate) for injectional solutions; and used sorbitol syrup, methylcellulose, glucose, sucrose syrup, hydroxyethylcellulose, food oil, glycerin, ethanol, water, emulsifiers (e.g., gum arabic and lecithin) and detergents (e.g., Tween and Span) for syrups and emulsions. For the solid forms, there can be used excipients (e.g., milk sugar, corn starch and mannitol), lubricants (calcium phosphate, magnesium stearate and talc), binders (e.g., sodium carboxymethylcellulose and hydroxypropylcellulose), disintegrators (e.g., crystalline cellulose, calcium carboxymethylcellulose) and fluid accelerators (e.g., light silicic anhydride).

The dosage of the compound of Formula (I) depends on the age of the patient, the kind and conditions of

the disease, but usually it is from 5 to 1000 mg in single or several divided doses per adult per day.

Then, the experiments are illustrated below in order to show the effects of the compounds of the present invention. The compound numbers in the following experiments are as defined in Examples described hereinafter.

Experiment 1 [Inhibition test of the radiofrequency decerebrate rigidity]

The rigidity animals were prepared according to the method of Ono et al. [Gen. Pharm., vol. 18, pages 57 - 59 (1987)].

Four male Wistar rats weighing 250 to 350 g were used for each group. The animals were anesthetized with ethyl ether and fixed on a brain stereotaxic apparatus to break the midbrain bilaterally (APO, V-3, L±1.5). Rigidity occurred gradually in the hindlimbs after discontinuation of ethyl ether anesthesia. The test drugs dissolved in propylene glycol were administered intravenously in a dose of 5 mg/kg (0.1 ml per 100 g of rat) to determine the inhibition time of the rigidity.

Results are shown in Table 1.

Table 1

| Test Drug | Inhibition Time (Minutes) |
|---|---|
| Compound 2 | 11.7 |
| Compound 4 | 37.0 |
| Compound 8 | 78.0 |
| Compound 11 | 24.0 |

Experiment 2 [Inhibition test of anemic decerebrate rigidity]

The rigidity animals were prepared according to the method of Fukuda et al. [Japanese J. Pharmacol., vol. 24, pages 810 - 813 (1974)].

Four male Wistar rats weighing 250 to 350 g were used for each group.

The animal was incised on the cervical region under the ethyl ether anesthesia, and the common carotid arteries were ligated bilaterally. A trephined opening was made in the occipital bone and the basilar artery was coagulated using a bipolar electro-coagulator. Marked rigidity occurred on the forelimbs after discontinuation of the ethyl ether anesthesia. The test drugs dissolved in polyethlene glycol 400 were each administered intravenously in a dose of 5 mg/kg (0.1 ml per 100 g of rat) to determine the inhibition time of the rigidity.

Results are shown in Table 2.

Table 2

| Test Drug | Inhibition Time (Minutes) |
|---|---|
| Compound 2 | 5.0 |
| Compound 8 | 17.7 |

Experiment 3 [Straub tail test]

The test was carried out according to the method of Ellis et al. [Neuropharmacology, vol. 13, pages 211 - 214 (1974)].

Five male ICR mice weighing 20 to 30 g were used for each group. The test drugs suspended in 0.4% aqueous carboxymethylcellulose solution were administered orally in a dose of 100 mg/kg (0.1 ml per 10 g of mouse). After 15 minutes, 15 mg/kg of morphine hydrochloride was administered subcutaneously, and after 30 minutes, the tail elevation was determined. The muscle relaxation activity was defined as positive when the angle of tail elevation from the horizontal was less than 45°. As a result, the inhibition ratio of Compounds 2, 4 and 8 were each 100%.

Experiment 4 [Acute toxicity test]

Ten male ICR mice weighing 25 to 34 g were used. Compound 4 suspended in 0.4 aqueous carboxymethylcellulose solution was each administered orally to mice in a volume of 0.1 ml per 10 g of mice. The survivals were observed 7 days after administration. No death occurred in the dose of 1 g/kg, and the $LD_{50}$ value proved to be more than 1 g/kg p.o.

The present invention is illustrated by the following examples in more detail. The compound numbers in Examples 3 to 7 are as defined in Examples 1 and 2.

Example 1

(1) To a solution of 20.0 g of 2-(2-fluorophenylthio)propionic acid in 200 ml of toluene was added 14.5 ml of thionyl chloride, and the mixture was stirred at 70°C for 5 hours. The toluene and the excess thionyl chloride were evaporated to dryness under reduced pressure to give 22 g of the crude acid chloride.

To a solution of the above crude compound in 200 ml of toluene was added dropwise with ice cooling with stirring a solution of 20.6 ml of cyclopropylamine in 50 ml of toluene, and the mixture was stirred at room temperature for 15 hours. The reaction solution was washed, in turn, with water, a saturated sodium bicarbonate aqueous solution, dilute hydrochloric acid and water and drided over anhydrous magnesium sulfate, and the toluene was evaporated under reduced pressure. The residue was recrystallized from ethyl acetate to give 21.0 g of N-cyclopropyl-2-(2-fluorophenylthio)propionamide as colorless needles.

m.p. 56 - 57°C

(2) To a solution of 2.39 g of N-cyclopropyl-2-(2-fluorophenylthio)propionamide in 100 ml of dichloromethane was added at room temperature with stirring a solution of 5.10 g of m-chloroperbenzoic acid (purity: 70%) in 130 ml of dichloromethane, and the mixture was stirred at room temperature for 24 hours, after which the dichloromethane was evaporated.

The resulting residue was dissolved in ethyl acetate, the solution was washed, in turn, with water, a saturated sodium bicarbonate aqueous solution and water and dried over anhydrous magnesium sulfate, and the ethyl acetate was evaporated. The residue was recrystallized from ethyl acetate to give 2.19 g of N-cyclopropyl-2-(2-fluorophenylsulfonyl)propionamide (Compound 1) as colorless needles.

m.p. 90 - 91°C.

Example 2

(1) To a solution of 21.7 g of 2-(2-chlorophenylthio)propionic acid in 200 ml of toluene was added under a nitrogen stream with ice cooling with stirring 13.9 ml of triethylamine and then 13.0 ml of isobutyl chlorocarbonate. The mixture was stirred at room temperature for 30 minutes. The reaction solution was again cooled on ice, and 7.6 ml of cyclopropylamine was added dropwise with stirring, after which the mixture was stirred at room temperature for 2 hours. Then, following a procedure similar to that of Example 1 (1), there was obtained 20.5 g of N-cyclopropyl-2-(2-chlorophenylthio)-propionamide.

m.p. 103 - 104°C.

(2) To 2.56 g of N-cyclopropyl-2-(2-chlorophenylthio)propionamide were added 30 ml of acetic acid and 5 ml of hydrogen peroxide, and the mixture was stirred at 100°C for an hour. The acetic acid and water were evaporated under reduced pressure, the residue was dissolved in ethyl acetate, washed, in turn, with water, a saturated sodium bicarbonate aqueous solution and water and dried over anhydrous magnesium sulfate, and the ethyl acetate was evaporated under reduced pressure. The residue was recrystallized from n-hexane - ethyl acetate to give 2.71 g of N-cyclopropyl-2-(2-chlorophenylsulfonyl)propionamide (Compound 2).

m.p. 94 - 95°C.

Following a procedure similar to that of Example 1 or 2 using the corresponding starting materials, there were obtained the compounds shown in Table 3.

Table 3

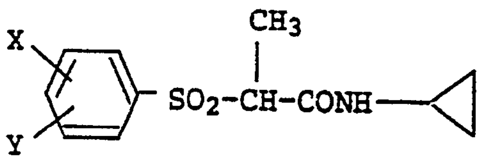

| Compound No. | X | Y | m.p. (°C) |
|---|---|---|---|
| 3 | 4 - Cl | H | 149 - 150 |
| 4 | 2 - Br | H | 83 - 84 |
| 5 | 3 - Br | H | 116 - 117 |
| 6 | 4 - Br | H | 161 - 162 |
| 7 | 2 - Cl | 3 - Cl | 148 - 149 |
| 8 | 2 - Cl | 4 - Cl | 96 - 97 |
| 9 | 2 - Cl | 5 - Cl | 164 - 165 |
| 10 | 2 - Cl | 6 - Cl | 138 - 139 |
| 11 | 4 - F | 2 - Cl | 122 - 123 |
| 12 | 4 - F | 3 - Cl | 162 - 163 |

Example 3 (Tablets)

| | |
|---|---|
| Compound 4 | 600 g |
| Crystalline cellulose | 120 g |
| Corn starch | 125 g |
| Hydroxypropylcellulose | 45 g |
| Magnesium stearate | 10 g |
| Total | 900 g |

The above components were mixed according to an ordinary manner and tableted to give 9 mm diameter tablets weighing 300 mg.

Example 4 (Cupseles)

| | |
|---|---|
| Compound 4 | 600 g |
| Crystalline cellulose | 150 g |
| Corn starch | 140 g |
| Magnecium stearate | 10 g |
| Total | 900 g |

The above components were mixed according to an ordinary manner, and each 300 mg of the mixture was filled into a No. 1 gelatin capsule.

Example 5 (Granules)

| | |
|---|---|
| Compound 8 | 200 g |
| Mannitol | 300 g |
| Corn starch | 450 g |
| Magnesium stearate | 10 g |
| Hydroxypropylcellulose | 50 g |
| Total | 1010 g |

Granules were prepared from the above components by a wet gramulation method.

Example 6 (Powders)

| | |
|---|---|
| Compound 4 | 200 g |
| lactose | 800 g |
| Total | 1000 g |

The above components were mixed uniformly according to an ordinary manner to give powders, each 1000 mg of which were filled into a pack.

Example 7 (Injectional solution)

Fifty g of Compound 4 was dissolved in 1000 ml of distilled water for injection and filled into 2 ml ampules according to an ordinary manner.

**Claims**

1. Sulfonylalkanoic acid amide derivatives of the formula:

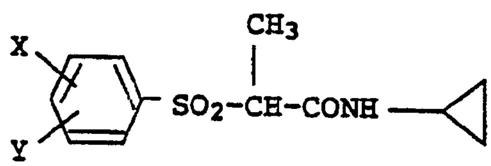

(I)

wherein X is a halogen atom; and Y is a hydrogen or a halogen atom.

2. A sulfonylalkanoic acid amide derivative as claimed in claim 1 which is N-cyclopropyl-2-(2-bromophenylsulfonyl)propionamide or N-cyclopropyl-2-(2,4-dichlorophenylsulfonyl)propionamide.

3. A pharmaceutical composition for relaxing muscle tone which comprises an sulfonylalkanoic acide amide derivative as claimed in claim 1 or claim 2, together with a pharmaceutically acceptable carrier.

4. Use of a sulfonylalkanoic acid amide derivative as claimed in claim 1 or claim 2 in themanufacture of a pharmaceutical composition for relaxing muscle tone.

5. A process for preparing a sulfonylalkanoic acid amide derivative of the formula:

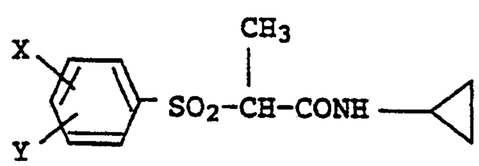

(I)

(wherein X is a halogen atom, and Y is a hydrogen or a halogen atom), which comprises reacting a sulfide compound of the formula

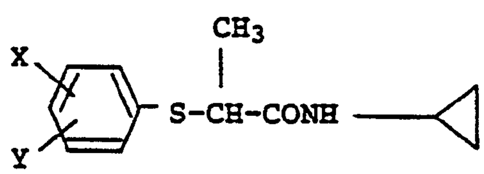

(III)

(wherein X and Y are as defined above) with an oxidizing agent.

6. A process as claimed in claim 5 in which the sulfonylalkanoic acid amide derivative is N-cyclopropyl-2-bromophenylsulfonyl)propionamide or N-cyclopropyl-2-(2,4-dichlorophenylsulfonyl)propionamide.

7. A method for relaxing muscle tone which comprises administering to a patient an effective amount of a sulfonylalkanoic acid amide derivative as claimed in claim 1 or claim 2.